# FASCICULE DE BREVET EUROPEEN

(11) **EP 1 117 684 B1**
(45) Date de publication et mention de la délivrance du brevet: **28.03.2012**
(21) Numéro de dépôt: 99946270.8
(22) Date de dépôt: 30.09.1999
(51) Int. Cl.: C07K 14/47, A61K 38/17, G01N 33/58, G01N 33/86

(54) **STRUCTURE CHIMIQUE AYANT UNE AFFINITE POUR UN PHOSPHOLIPIDE, ET COMPOSE DE MARQUAGE, TROUSSE DE DIAGNOSTIC, ET MEDICAMENT COMPRENANT CETTE STRUCTURE**
CHEMISCHE STRUKTUR MIT AFFINITÄT FÜR EIN PHOSPHOLIPID, SOWIE MARKIERUNGSVERBINDUNG, DIAGNOSEKIT UND ARZNEIMITTEL, WELCHES DIESE STRUKTUR ENTHÄLT
CHEMICAL STRUCTURE WITH AFFINITY FOR A PHOSPHOLIPID, AND MARKER COMPOUND, DIAGNOSIS KIT, AND MEDICINE COMPRISING SAID STRUCTURE

(30) Priorité: 02.10.1998 FR 9812366
(43) Date de publication de la demande: 25.07.2001
(73) Titulaire: Commissariat à l'Énergie Atomique et aux Énergies Alternatives, 75015 Paris (FR); Université Pierre et Marie Curie (Paris VI), 75252 Paris Cedex 05 (FR)
(72) Inventeur: SANSON, Alain, F-91940 Gometz le Chatel (FR); RUSSO-MARIE, Françoise, F-92310 Sèvres (FR); NEUMANN, Jean-Michel, F-91190 Gif sur Yvette (FR); CORDIER-OCHSENBEIN, Françoise, F-75005 Paris (FR); GUEROIS, Raphael, F-75005 Paris (FR)
(74) Mandataire: Audier, Philippe André
(86) Numéro de dépôt international: PCT/FR1999/002329
(87) Numéro de publication internationale: WO 2000/020453

(56) Documents cités:
- WO-A-91/09953
- WO-A-92/19279
- US-A- 5 627 036
- F. CORDIER-OCHSENBEIN ET AL.: "Exploring the folding pathways of annexin I, a multidomain protein. I." J. MOL. BIOL., vol. 279, juin 1998 (1998-06), pages 1163-1175, XP002107103
- K. SANO ET AL.: "Isolation and sequence of a c-DNA clone for the rat pulmonary surfactant-associated protein (PSP-A)" BIOCHEMICAL AND BIOPHYSICAL RESEARCH COMMUNICATIONS., vol. 144, no. 1, 1987, pages 367-374, XP002107104 ORLANDO, FL US
- F. MACQUAIRE ET AL: "Proton NMR Conformational study of an annexin I fragment" BIOCHEMISTRY, vol. 32, 1993, pages 7244-7254, XP002107105
- J.D. ERNST ET AL: "Annexins possess functionality distinguishable Ca2+ and phospholipid binding domains" BIOCHEMICAL AND BIOPHYSICAL RESEARCH COMMUNICATIONS., vol. 200, no. 2, 1994, pages 867-876, XP002107106 ORLANDO, FL US
- R. HUBER ET AL: "The calcium binding sites in human annexin V by crystal structure analysis at 2.0 A resolution" FEBS LETTER, vol. 275, no. 1,2, 1990, pages 15-21, XP002107107

## Description

### Domaine technique

La présente invention se rapporte à une structure chimique ayant une affinité pour un phospholipide ainsi qu'à une molécule de détection, à un conjugué et à une composition pharmaceutique comprenant ladite structure.

De manière générale, la structure chimique de la présente invention est utile pour la reconnaissance spécifique de vecteurs lipidiques. Elle est utilisable pour l'ingénierie et la création de composés de reconnaissance et de séquestration de lipides notamment de lipides chargés négativement, tels que la phosphatidylsérine et/ou l'acide phosphatidique.

Ces lipides jouent un rôle important notamment dans la signalisation cellulaire et peuvent être présents à la surface externe des membranes des cellules et/ou circuler dans le milieu sanguin à la suite d'événements pathologiques très divers.

Divers événements cellulaires aboutissent à l'apparition de phosphatidylsérine (PS) à la surface externe des cellules, ces événements peuvent résulter soit d'une altération fortuite ou pathologique de la cellule, soit d'un événement cellulaire programmé telle que la mort cellulaire ou apoptose. L'apparition de PS à la surface externe des cellules constitue donc un "message primaire" important témoignant de l'existence d'un dysfonctionnement. Dans le cas du processus de coagulation sanguine, le mécanisme est bien décrit : l'altération des cellules endothéliales des vaisseaux sanguins, soit pour des raisons accidentelles, soit pour des raisons pathologiques plus complexes, provoque l'apparition de ce message PS à la surface externe des cellules en contact avec le milieu sanguin. Ce message est immédiatement reconnu par certaines protéines circulantes qui déclenchent alors une cascade d'événements aboutissant au phénomène de coagulation sanguine bien connu.

L'invention tire profit de la propriété de la structure qu'elle fournit de se lier, en présence ou non de calcium, aux lipides et notamment ceux chargés négativement, pour la mise en point de composés utilisables comme outils de recherche, de diagnostic et de thérapeutique dans le domaine de la reconnaissance des effecteurs lipidiques en général et de la détection de l'apoptose, des troubles de la coagulation sanguine, du choc septique et des pathologies inflammatoires aiguës en particulier.

Concernant la recherche et le diagnostic, la structure de l'invention peut par exemple être couplée à des molécules de détection, par exemple à une molécule fluorescente, au complexe avidine-biotine, à un radioélément à vie courte, ou à un composé paramagnétique. Avec ces molécules de détection, il est possible par exemple de détecter des cellules apoptotiques ou de reconnaître des microdomaines membranaires chargés négativement.

La structure de la présente invention peut donc être utilisée pour une détection "*in vitro"* de pathologies impliquant l'apparition de charges négatives à la surface des cellules et la libération dans le sang de microvésicules.

La structure de la présente invention peut également être utilisée lorsqu'elle est couplée par exemple à un radioélément à vie courte, pour une détection *"in vivo*" de zones thrombotiques lors d'accidents vasculaires de toute sorte, en particulier cérébraux, en utilisant des systèmes d'imagerie. Cette structure peut par ailleurs être utilisée lorsqu'elle est couplée à un composé paramagnétique tel qu'un complexe gadolinium pour une détection "in vivo" de zones thrombotiques, en particulier cérébrales, en utilisant l'imagerie par résonance magnétique (IRM).

Concernant la thérapeutique, de manière générale, la structure de la présente invention peut être utilisée seule ou couplée à une molécule thérapeutique pour préparer un médicament utilisable par exemple par voie orale. Un tel médicament peut par exemple être utilisé pour le ciblage de cette molécule vers des zones présentant des charges négatives telles que des tumeurs présentant des foyers de cellules apoptotiques ou des tumeurs inflammatoires.

La structure dé la présente invention peut par exemple être couplée à des molécules à action thrombolytique pour préparer un médicament qui peut être utilisé par exemple par voie orale en tant qu'anti-coagulant dans le traitement et la prophylaxie de la thrombose, ou pour préparer une molécule recouvrant tous les biomatériaux thrombogènes. La structure de la présente invention peut donc être utilisée pour le ciblage des molécules thrombolytiques au site du thrombus ou vers les zones thrombogènes.

Dans un autre exemple d'application de la présente invention, la structure de l'invention peut être seule ou couplée à une molécule anti-inflammatoire pour préparer un médicament qui peut être utilisé par voie orale, par exemple dans des pathologies aiguës comme l'asthme, la rectocolite hémoragique (RCH), le Crohn, le choc septique, les maladie du collagène et de l'arthrite.

### Etat de la technique

Une famille de protéines, appelées annexines, ont été décrites dans l'art antérieur comme présentant un ancrage fonctionnel réversible à la membrane cellulaire, régulé par la concentration en calcium et la présence de phospholipides anioniques. Les annexines constituent une famille de protéines exprimées dans des tissus très divers, aussi bien chez les animaux que chez les plantes. Il semble qu'elles ne sont ni exprimées chez la bactérie, ni chez la levure.

La structure des annexines comporte quatre domaines d'environ 70 acides aminés, ou résidus, très moyennement homologues en séquence mais de topologie quasiment identique.

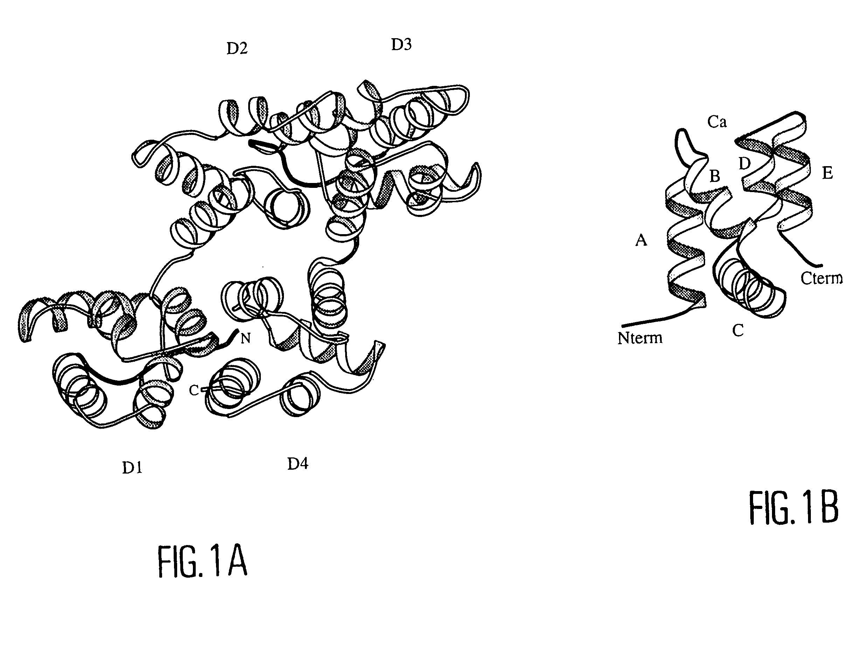

La figure 1A en annexe est un schéma de la topologie générale d'une annexine et la figure 1B en annexe est un schéma de la topologie d'un domaine de l'annexine portant un site calcium. Sur la figure 1A, C représente l'extrémité C-terminale de cette protéine, N représente l'extrémité N-terminale de cette protéine. Les domaines, notés D1 à D4, sont associés en deux modules, l'un covalent D2D3, l'autre non covalent D1D4. Sur la figure 1B, A représente une première hélice α, B représente une deuxième hélice α, C représente une troisième hélice α, D représente une quatrième hélice α, E représente une cinquième hélice α, et Ca représente l'atome de calcium. L'association de ces hélices constitue la structure consensus pour un domaine d'annexine.

A l'heure actuelle, leurs rôles biologiques demeurent encore mal définis.

Dans le document WO 92/19279, J. TAIT décrit des conjugués ayant une affinité pour des phospholipides. Il décrit en particulier l'utilisation de l'annexine, en particulier de l'annexine V, pour fabriquer un conjugué actif utilisable en tant qu'agent thrombolytique.

Malheureusement, le conjugué décrit dans ce document est préparé à partir de l'annexine entière par un procédé de recombinaison génétique. De ce fait, il apparaît de nombreux inconvénients qui sont notamment un rendement faible, un coût de fabrication élevé, et l'obtention d'un conjugué fragile du fait de sa partie protéique complexe.

### Exposé de l'invention

La présente invention a précisément pour but de fournir une structure chimique peptidique ayant affinité spécifique avec un phospholipide telle que définie dans les revendications 1 à 6 structure chimique de l'invention présente notamment l'avantage d'être stable chimiquement et de pouvoir être fabriquée de manière reproductible, avec un rendement élevé et un coût de fabrication très réduit par rapport aux composés de l'art antérieur

La structure peptidique de la présente invention dérive d'une structure qui comprend au moins une plate-forme chimique U, V, W, X, Y comportant six résidus RL1, RL2, RL3, RL4, RL5, RL6 supportant un ensemble de fonctions chimiques pouvant se lier audit phospholipide appelées L1, L2, L3, L4, L5, L6 respectivement, ces fonctions chimiques définissant au moins en partie l'affinité de ladite structure pour ledit phospholipide, ladite structure ayant une des constructions (I), (II) et (III) suivantes : 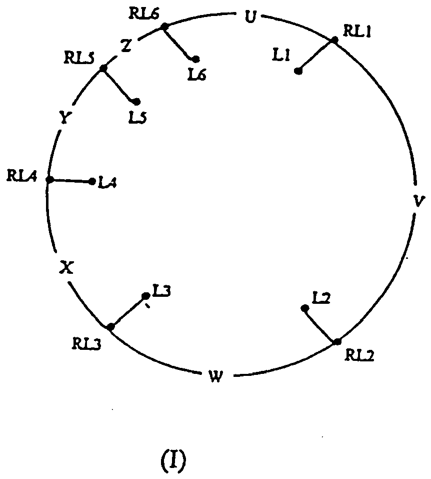 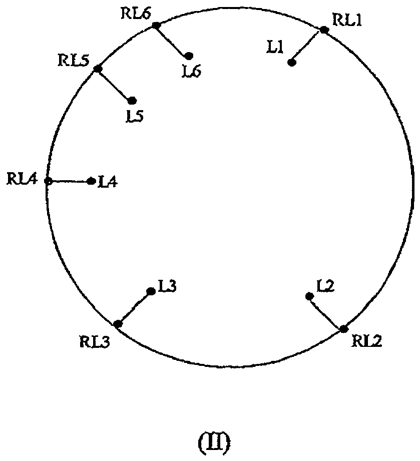 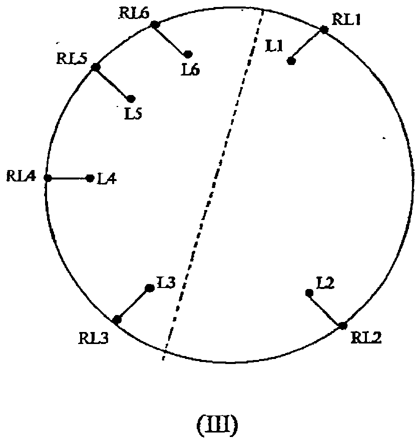 dans lesquelles U, U¹, U², V, W, W¹, W² X, Y, Z sont indépendamment un acide aminé naturel ou non naturel, un peptide constitué d'acides aminés naturels ou non naturels, une chaîne carbonée, ou un ou des groupe (s) cyclique (s) carboné (s),
dans lesquelles RL1 à RL6 sont choisis parmi des molécules présentant les fonctions chimiques de liaison L1 à L6 respectivement, lesdites fonctions chimiques comprenant soit au moins une charge positive et donneuse de liaison hydrogène, soit au moins une charge négative et acceptrice de liaison hydrogène, et
dans lesquelles U, U¹,U², V, W, X, Y et Z sont tels que RL6 et RL1 sont distants de 0,65 à 0,95 nm, L6 et L1 sont distants de 0,65 à 0,9 nm, RL1 et RL2 sont distants de 0,45 à 0,65 nm, L1 et L2 sont distants de 0,4 à 0,55 nm, RL2 et RL3 sont distants de 0,5 à 1,05 nm, L2 et L3 sont distants de 0,4 à 0,6 nm, RL3 et RL4 sont distants de 0,5 à 0,8 nm, L3 et L4 sont distants de 0,35 à 0,5 nm, RL4 et RL5 sont distants de 0,45 à 0,75 nm et L4 et L5 sont distants de 0,4 à 0,55 nm, RL5 et RL6 sont distants de 0,4 à 1,2 nm, L5 et L6 sont distants de 0,4 à 0,6 nm.

Dans la structure de construction (I), (II) ou (III), L1, L2, L3 et L6 peuvent présenter chacune au moins une charge positive et donneuse de liaison hydrogène, et L4 et L5 peuvent présenter chacune au moins une charge négative et acceptrice de liaison hydrogène.

Dans la structure de construction (I), (II) ou (III), U, V, W, X , Y et Z peuvent être des peptides constitués d'acides aminés naturels ou non naturels, et RL1 à RL6 sont des acides aminés choisis dans un ensemble comprenant Lys, Arg, Orn, Ser, Thr, Asp et Glu, ou des analogues de ceux-ci, L1 à L6 étant les fonctions chargées des chaînes latérales desdits acides aminés.

Dans la structure de construction (I), (II) ou (III), RL1 à RL6 peuvent être disposés dans l'espace formé par U, V, W, X, Y, Z de manière à ce que les fonctions chimiques de liaison L1 à L6 respectivement de leur chaîne latérales soient directement accessibles au phospholipide.

La structure de construction (I), (II) ou (III) peut comprendre en outre un site calcium où l'ion calcium complexé par ce site constitue un des ligands du phospholipide.

La présente invention est également basée sur une structure chimique qui est caractérisé en ce qu'elle comprend au moins une plate-forme chimique a, a', b, b', c, d, e, f, g, h, i, j, k, 1 comportant 11 résidus RL1, RL2, RL3, RL4, RL5, RL6, RCa1, RCa2, RCa3, RCa4 et RCa5 supportant un ensemble de fonctions chimiques pouvant se lier audit phospholipide appelées L1, L2, L3, L4, L5, L6 respectivement, et un ensemble de fonctions chimiques de liaison à un atome de calcium appelées LCa1, LCa2, LCa3, LCa4, LCa5 respectivement, ces fonctions chimiques RL1 à RCa5 définissant au moins en partie l'affinité de ladite structure pour ledit phospholipide, ladite structure ayant une des constructions (IV), (V) et (VI) suivantes : 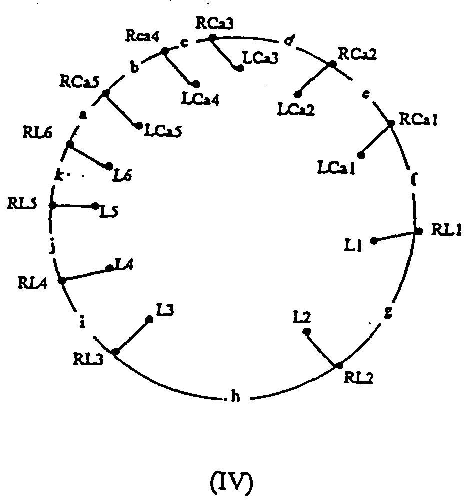 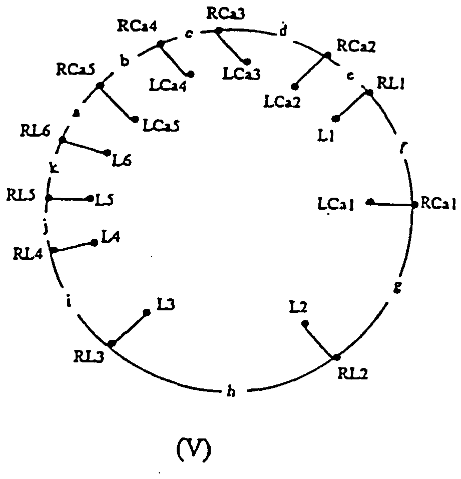 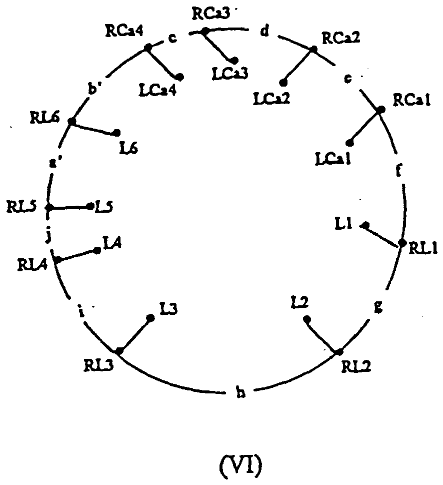 dans lesquelles a, a', b, b', c, d, e, f, g, h, i, j, k, 1 sont indépendamment un acide aminé naturel ou non naturel, un peptide constitué d'acides aminés naturels ou non naturels, une chaîne carbonée, ou un ou des groupe(s) cyclique(s) carboné(s),
dans lequel RL1 à RL6 et RCa1 à RCa5 sont choisis parmi des molécules présentant les fonctions chimiques de liaison L1 à L6 et LCa1 à LCa5 respectivement, lesdites fonctions chimiques L1 à L6 comprenant soit au moins une charge positive et donneuse de liaison hydrogène, soit au moins une charge négative et acceptrice de liaison hydrogène, lesdites fonctions chimiques LCa1 à LCa5 comprenant un atome d'oxygène, et
dans lesquelles a dans les structures de construction (IV) et (V) est tel que RL6 et RCa5 sont distants de 0 à 0,35 nm et tel que L6 et LCa5 sont distants de 0 à 0,3 nm, b dans les structures de construction (IV) et (V) est tel que RCa5 et RCa4 sont distants de 0 à 0,35 nm et tel que LCa5 et LCa4 sont distants de 0,2 à 0,3 nm, b' dans la structure de construction (VI) est tel que RL6 et RCa4 sont distants de 0 à 0,35 nm et tel que L6 et LCa4 sont distants de 0 à 0,35 nm, c et d sont tels que RCa4 et RCa3 sont distants de 0,5 à 0,9 nm, LCa4 et LCa3 sont distants de 0,2 à 0,4 nm, RCa3 et RCa2 sont distants de 0,35 à 0,6 nm, et LCa3 et LCa2 sont distants de 0,22 à 0,3 nm, e, f, g, dans les structures de construction (IV), (V), (VI) sont tels que RL1 et RL2 sont distants de 0,45 à 0,65 nm, RCa1 à RCa2 sont distants de 0,4 à 0,55 nm, L1 et L2 sont distants de 0,4 à 0,55 nm et LCa1 et LCa2 sont distants de 0,3 à 0,4 nm, h, i, j et k sont tels que RL2 et RL3 sont distants de 0,5 à 1,05 nm, L2 et L3 sont distants de 0,4 à 0,6 nm, RL3 et RL4 sont distants de 0,5 à 0,8 nm, L3 et L4 sont distants de 0,35 à 0,5 nm, RL4 et RL5 sont distants de 0,45 à 0,75 nm, L4 et L5 sont distants de 0,4 à 0,55 nm, RL5 et RL6 sont distants de 0,4 à 1,2 nm, et L5 et L6 sont distants de 0,4 à 0,6 nm, a' dans la structure de construction (VI) est tel que RL5 et RL6 sont distants de 0,4 à 1,2 nm et tel que L5 et L6 sont distants de 0,4 à 0,6 nm, et b' dans la structure de construction (VI) est tel que RL6 et RCa4 sont distants de 0 à 0,35 nm et tel que L6 et LCa4 sont distants de 0 à 0,35 nm, la structure pouvant être soit fermée, soit ouverte au niveau de a et/ou de h.

Lorsque les distances précédentes a, b, b' sont indiquées comme pouvant être nulles, on sous-entend que les deux ensembles (RL6-L6 et Rca5-Lca5) et/ou les deux ensembles (Rca4-Lca4 et Rca5-Lca5) et/ou les deux ensembles (RL6-L6 et Rca4-Lca4) constituent séparément un seul et même ensemble.

Les plates-formes sont constituées d'un ensemble de groupes chimiques structuraux pouvant comprendra un nombre de groupes acycliques suffisants pour assurer une rigidité compatible avec l'affinité au phospholipide.

Les distances mesurées lorsque les RL et les RCa sont des acides aminés, peuvent être mesurées entre les carbones α de ces acides aminés dans les structures (I) à (VI) précitées.

Ces structures peuvent être synthétisées par les procédés classiques de synthèse de la chimie organique et de la chimie des protéine, par recombinaison génétique, par génie génétique, etc...

Des exemples de telles structures sont données notamment dans "Discovery of Séquence-Sélective Peptide Binding by Synthetic Receptors Using Encoded Combinatorial Libraries", W.C. Still, Acc. Chem. Res., 1996, 29. 155-163 et dans "Toward Synthetic Adrénaline Receptors : Strong, Selectrive and Biomimetic Recognition of Biologically Active Amino Alcohols by Bisphosphonate Receptors Molecules", T. Shrader, J. Org. Chem., 1998, 63, 264-272.

Dans la structure de construction (IV), (V) ou (VI), L1, L2, L3 et L6 peuvent présenter chacune au moins une charge positive et donneuse de liaison hydrogrène, et L4, L5, LCa5, LCa4, LCa3, LCa2 et LCa1 peuvent présenter chacune au moins une charge négative et acceptrice de liaison hydrogène.

Dans la structure de construction (I), (II), (III), RL1, RL2, RL3 et RL6 peuvent être choisis indépendamment parmi. Arg, Lys. Orn ; RL4 peut être choisi indépendamment parmi Asp ou Glu ; et RL5 peut être choisi indépendamment parmi Ser, Thr. Asp ou Glu, les chaînes latérales de ces acides aminés présentant les fonctions chimiques de liaison au phospholipides L1 à L6 respectivement.

Dans la structure de construction (rv), (V) ou (VI), a ou a', b ou b', c, d, e, f, g, h, i, j, k peuvent être des peptides constitués d'acides aminés naturels ou non naturels, et RL1 à RL5 peuvent être des acides aminés choisis dans un ensemble comprenant Lys, Arg, Orn, Ser, Thr, Asp et Glu, ou des analogues de ceux-ci, RL6 peut être Asp ou Glu ou des analogues de ceux-ci, L1 à L6 et LCa1 à LCa5 peuvent être les fonctions chargées des chaînes latérales desdits acides aminés, et RCa1 à RCa5 peuvent être des acides aminés naturels ou non naturels.

Dans la structure de construction (IV), (V) ou (VI) . Les carbones RL1 à RL6 et RCa1 à RCa2 peuvent être disposés dans l'espace formé par a, b, c, d, e, f, g, h, i, j et k de manière à ce que les fonctions chimiques de liaisons L1 à L6 respectivement et les charges positives du calcium lorsque ce dernier est lié aux fonctions de liaison Loca1 à LCa5 soient directement accessibles au phospholipide.

Dans la structure de construction (I), (II), (III), (IV), (V) ou (VI), au moins une partie de la plate-forme peut être une partie d'un domaine de l'annexine ou d'un domaine modifié de l'annexine, comprenant au moins un desdits résidus ligand RL1 à RL6 présentant lesdites fonctions L1 à L6 respectivement de liaison au phospholipide.

Dans la structure de construction (I), (II), (III), (IV), (V) ou (VI), la plate-forme peut être une partie d'un domaine de l'annexine ou d'un domaine modifiée de l'annexine, ladite partie du domaine de l'annexine comprenant lesdits résidus ligands RL1 à RL6 présentant lesdites fonctions L1 à L6 respectivement.

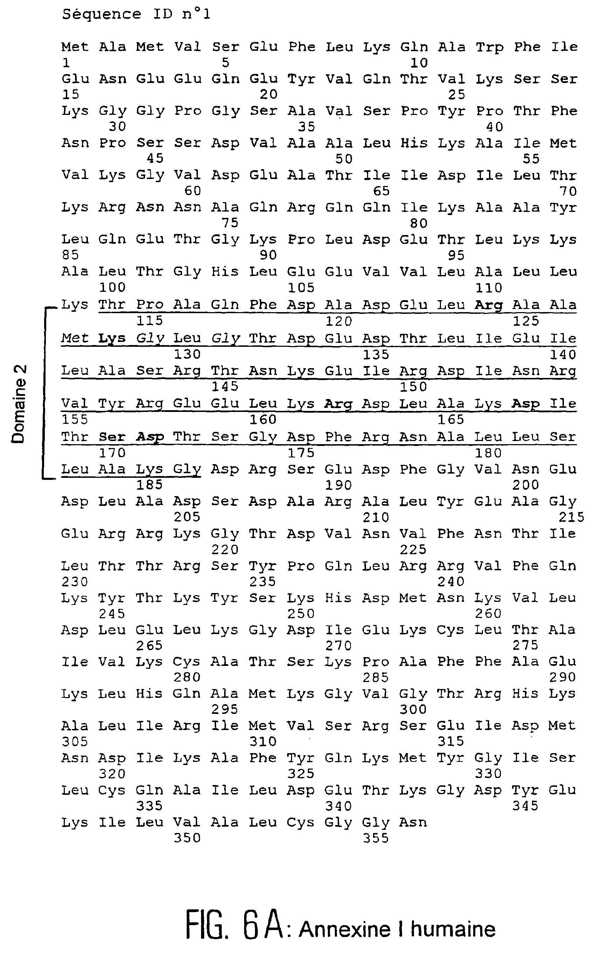

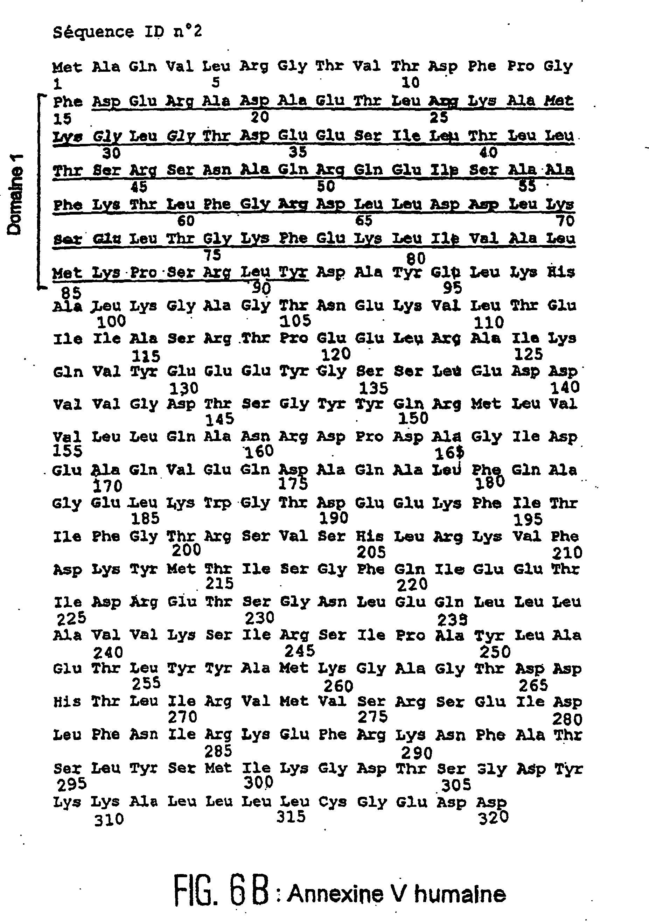

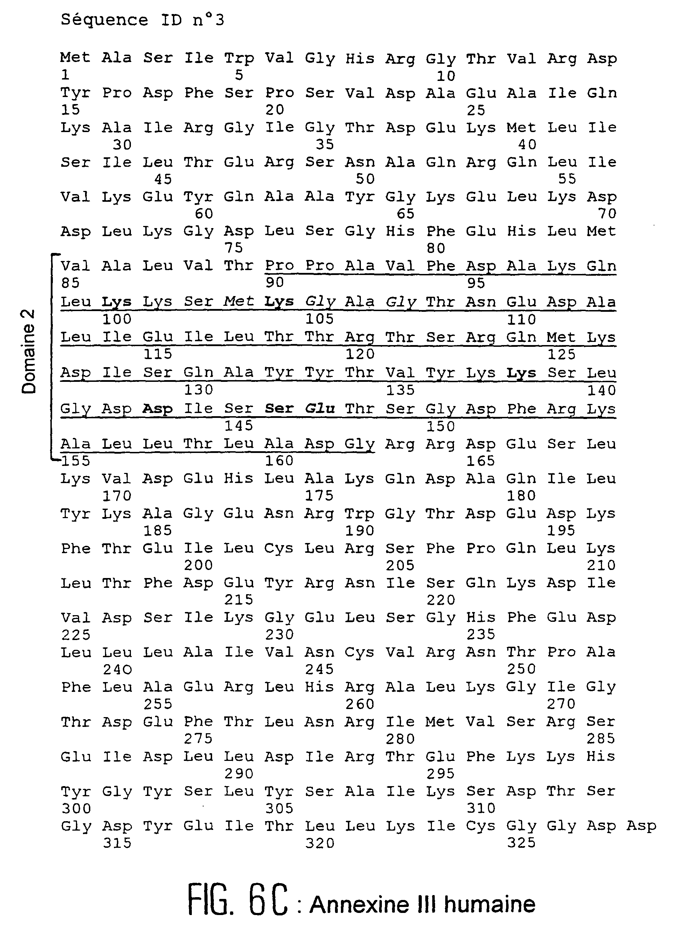

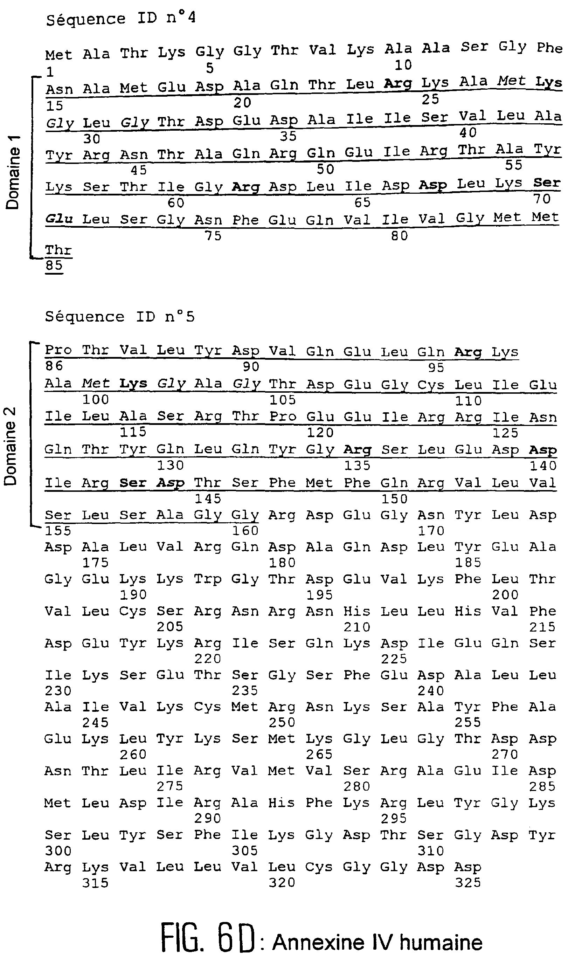

Selon l'invention, le domaine de l'annexine est choisi parmi le domaine 1 de l'annexine V présenté sur la figure 6b, le domaine 2 de l'annexine I présenté sur la figure 6a, le domaine 2 de l'annexine III présenté sur la figure 6c et le domaine 1 et 2 de l'annexine IV présentés sur la figure 6d.

Selon l'invention, les résidus ligands RL1 à RL6 peuvent être respectivement soit, les résidus Arg25, Lys29, Arg63, Asp68, Ser71 et Glu 72 du domaine 1 de l'annexine V présenté sur la figure 6b, soit les résidus Arg124, Lys128, Arg162, Asp167, Ser170 et Asp171 du domaine 2 de l'annexine I présenté sur la figure 6a, soit les résidus Lys100, Lys104, Lys138, Asp143, Ser146 et Glu147 du domaine 2 de l'annexine III présenté sur la figure 6c, soit les résidus Arg97, Lys101, Arg135, Asp140, Ser143 et Asp144 du domaine 2 de l'annexine IV présenté sur la figure 6d, soit les résidus Arg24, Lys28, Arg62, Asp67, Ser70 et Glu71 du domaine 1 de l'annexine IV présenté sur la figure 6d.

La présente invention décrit également une structure chimique ayant une affinité pour un phospholipide caractérisée en ce qu'elle, comprend une molécule de formule (VII) suivante :

RL1-N¹-RL2-M-RL3-N²-RL4-N³-RL5-RL6 (VII)

dans laquelle N¹ à N³ représentent chacun indépendamment de 1 à 4 des acides aminés choisis indépendamment, naturels ou non naturels, et dans laquelle M est un peptide constitué de 1 à 100 acides aminés naturels ou non naturels ;
dans laquelle RL1, RL2, RL3 et RL6 sont choisis indépendamment parmi Lys, Arg ou Orn ; RL4 est choisi indépendamment parmi Asp ou Glu ; et RL5 est choisi indépendamment parmi Ser, Thr, Asp ou Glu, ladite structure étant linéaire ou cyclique.

Selon l'invention, N¹ peut représenter trois acides aminés, N² peut représenter quatre acides aminés, et N³ peut représenter deux acides aminés dans la structure de formule VII.

Dans la structure selon l'invention, M est un peptide constitué de 33 acides aminés naturels ou non naturels.

Selon l'invention, la structure de formule (VII) peut comprendre une séquence peptidique choisie parmi la séquence peptidique allant de Arg124 à Ser171 dans la séquence ID n°1 présentée sur la figure 6a, la séquence peptidique allant de Arg25 à Glu72 dans la séquence ID n°2 présentée sur la figure 6b, la séquence peptidique allant de Lys100 à Glu147 dans la séquence ID n°3 présentée sur la figure 6c, la séquence allant de Arg24 à Glu71 dans la séquence ID n°4 présentée sur la figure 6d, la séquence allant de Arg97 à Asp144 dans la séquence ID n°5 présentée sur la figure 6d, ou une séquence modifiée de ces séquences pourvu que RL1, RL2, RL3 et RL6 soient choisis indépendamment parmi Lys, Arg ou Orn ;
RL4 choisi indépendamment parmi Asp ou Glu, et RL5 est choisi indépendamment parmi Ser, Thr, Asp ou Glu.

La présente invention fournit également une structure chimique ayant une affinité pour un phospholipide, comprenant au moins une partie d'une séquence peptidique choisie parmi la séquence ID n°1 présentée sur la figure 6a, la séquence ID n°2 présentée sur la figure 6b, la séquence ID n°3 présentée sur la figure 6c, et les séquences ID n°4 et ID n°5 présentées sur la figure 6d, ou une séquence modifiée de celle-ci.

La présente invention fournit également une structure chimique ayant une affinité pour un phospholipide chargé négativement, comprenant une séquence peptidique cyclique de formule (VIII) suivante : 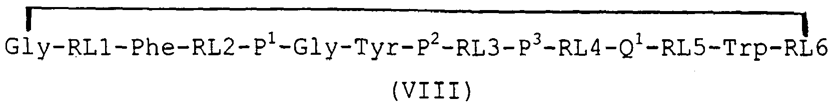 dans laquelle RL1 et RL6 sont choisis indépendamment parmi Lys, Orn et Arg ; RL2 et RL3 sont Arg ; RL4 et RL5 sont choisis indépendamment parmi Asp et Glu ;
dans laquelle P¹, P² et P³ sont choisis indépendamment parmi Ser et Thr ; dans laquelle Q¹ est choisi parmi Gly et Met.

Les structures chimiques précitées peuvent comprendre en outre un site calcium où l'ion calcium complexé par ce site constitue un des ligands du phospholipide chargé négativement. Le site calcium peut être par exemple un site calcium analogue à celui des annexines ou des phospholipides A2. Ces sites calcium sont connus par l'homme du métier.

Selon l'invention, toutes les structures chimiques précitées peuvent avoir une affinité pour un phospholipide choisi parmi une phosphatidylsérine, une phosphatidyléthanolamine, une phosphatidylinositol, un acide phosphatidique, et un cardiolipide, la ou les chaîne (s) lipidique (s) des phospholipides peuvent par exemple comprendre de 4 à 23 atomes de carbone. Par exemple, le phospholipide peut posséder une chaîne d'acide arachidonique, par exemple pour la phosphatidylsérine.

La présente invention fournit également un assemblage chimique ayant une affinité pour un phospholipide, comprenant au moins deux des structures chimiques de la présente invention, identiques ou différentes, lesdites structures étant liées.

Par exemple, dans un assemblage chimique de la présente invention, au moins une des structures chimiques peut être une des structures chimiques peptidiques précédemment décrites.

Les assemblages selon l'invention peuvent donc être composés par exemple de structures identiques ou différentes. Par exemple, l'assemblage peut être un assemblage covalent convenable de deux structures selon l'invention, par exemple des domaines 1 et 4 selon l'invention d'une même annexine. Cet assemblage peut par exemple comporter un domaine 4 selon l'invention modifié par génie génétique dans le but d'introduire un site calcium et phospholipidique identique à celui du domaine 1 de l'invention.

Ces domaines peuvent par exemple provenir des annexines I et V.

Ces assemblages peuvent avoir notamment pour but d'augmenter l'affinité des structures de la présente invention, pour le phospholipide, par exemple pour un phospholipide chargé négativement. Ils peuvent être réalisés par exemple par insertion d'un lien peptidique flexible, par exemple poly-glycine, entre les structures chimiques de l'invention.

Les structures et assemblages de la présente invention présentent une affinité pour les phospholipides, et notamment pour ceux chargés négativement, meilleure que 0,1 µM. Ils peuvent comprendre une partie d'une annexine ou l'un de ses dérivés. Cette annexine peut être une annexine naturelle ou modifiée par les moyens classiques de la chimie ou du génie génétique.

La présente invention fournit également un procédé de fabrication d'une structure chimique comprenant les étapes de préparation d'un cDNA comprenant une séquence de base codant pour ladite structure chimique, d'insertion du cDNA dans un vecteur d'expression approprié, de transformation d'une cellule hôte appropriée pour une réplication du plasmide et la fabrication de ladite structure par traduction dudit cDNA.

Selon l'invention, dans ce procédé, le vecteur peut être un plasmide, par exemple le vecteur pGEX-2T.

Dans le procédé selon l'invention, la cellule hôte appropriée peut être par exemple *E*. *Coli.*

Par exemple, pour la fabrication de la structure selon l'invention, on peut partir du domaine 1 de l'annexine I et modifier la séquence de telle manière que les résidus RL définis précédemment et éventuellement les résidus RCa apparaissent dans la séquence. Ainsi, par des procédés classiques de génie génétique, on peut fabriquer un cDNA codant pour la séquence modifiée et obtenir très facilement la structure de la présente invention. La structure selon l'invention, lorsqu'elle présente au moins une partie peptidique peut également être fabriquée par un procédé classique de synthèse chimique en phase solide.

Un exemple de modification de la séquence du domaine 1 de l'invention de l'annexine I peut consister à remplacer His52 par Arg, Met56 par Lys ou Arg, Val57 par Gly, Val60 par Thr, éventuellement Lys90 par Arg, Thr95 par Asp, Lys98 par Ser ou Thr, et Ala99 par Asp ou Glu. Ces modifications peuvent être faites sur d'autres domaines également.

Ces modifications peuvent avoir notamment pour rôle d'augmenter la stabilité générale de la structure ou du domaine vis-à-vis de la température, du pH, et des conditions ioniques du milieu d'utilisation ; de diminuer ses propriétés éventuelles de toxicité générale envers l'organisme humain ; d'augmenter son affinité pour les phospholipides chargés négativement ; et d'augmenter son affinité générale pour les membranes cellulaires.

Selon l'invention, la modification d'un domaine peut également avoir pour rôle de développer l'affinité de la structure pour un phospholipide, par exemple chargé négativement ; et même de retrouver une affinité au moins égale à celle que possède l'annexine dite sauvage, en l'absence de calcium.

La modification peut par exemple porter sur le résidu dit résidu bidentate Asp ou Glu de calcium (RL6) du ou des domaines portant un site phosphatidylsérine, pour les remplacer par l'un des résidus Lys ou Orn.

Une autre modification, par exemple du domaine 1 de l'annexine V peut consister à remplacer Glu 72 par Lys ou Orn, et/ou Thr 33 par Lys ou Orn.

Selon l'invention, la structure chimique ou l'assemblage de la présente invention peut être utilisée pour préparer un médicament.

Par exemple, le médicament peut être choisi parmi un médicament destiné au traitement d'une thrombose, un médicament destiné au traitement d'une tumeur, un médicament ayant une action anti-inflammatoire.

Selon l'invention, la structure ou l'assemblage chimique selon l'invention peut être couplé à une molécule de marquage pour former un composé de marquage.

Selon l'invention, la molécule de marquage peut être choisie par exemple parmi une molécule fluorescente, le complexe avidine-biotine, un radioélément, et un composé paramagnétique.

La présente invention fournit aussi une trousse de diagnostic comprenant une structure ou un assemblage précité.

Cette trousse de diagnostic peut par exemple comprendre en outre un réactif adéquat permettant de détecter ladite molécule de marquage.

La présente invention fournit également une trousse d'analyse et de détection de charges négatives à la surface de cellules, caractérisée en ce qu'elle comprend une structure ou un assemblage chimique de la présente invention.

La présente invention fournit également une trousse d'analyse et de détection de microvésicules dans le sang, caractérisée en ce qu'elle comprend une structure ou un assemblage chimique de la présente invention couplé à un marqueur.

D'autres avantages et caractéristiques de la présente invention apparaîtront encore à la lecture des exemples illustratifs et non limitatifs qui suivent, en référence aux figures en annexe.

### Brève description des figures

- la figure 1A est une représentation schématique de la structure générale des annexines ;
- la figure 1B est une représentation schématique de la structure d'un domaine d'une annexine comportant un site calcium ;
- la figure 2 est un schéma illustrant l'insertion dans un vecteur PGEX-2T du cDNA codant pour la structure chimique de la présente invention pour produire ledit composé par génie génétique ;
- la figure 3 est une représentation schématique d'un spectre RMN ¹H du domaine 1 de la présente invention de l'annexine I présentant la région des aliphatiques ;
- la figure 4 est une représentation graphique de la dénaturation du domaine 1 de la présente invention de l'annexine I par le chlorure de guanidinium ;
- la figure 5 est une représentation graphique de la dénaturation thermique du domaine 1 de la présente invention de l'annexine I ;
- la figure 6a représente la séquence de l'annexine I, notée séquence ID n°1, dans laquelle la séquence du domaine 2 de la présente invention a été soulignée ;
- la figure 6b représente la séquence de l'annexine V, notée séquence ID n°2, dans laquelle la séquence du domaine 1 de la présente invention a été soulignée ;
- la figure 6c représente la séquence de l'annexine III, notée séquence ID n°3, dans laquelle la séquence du domaine 2 de la présente invention a été soulignée ;
- la figure 6d représente la séquence de l'annexine IV, notée séquence ID n°4 et séquence ID n°5, dans laquelle la séquence des domaines 1 et 2 de la présente invention ont été soulignées ;
- la figure 7 est une représentation schématique de la structure de construction (I) de la présente invention liée à une molécule de phosphatidylsérine mettant en évidence les interactions entre les fonctions de liaison L1 à L6 de la structure de construction (I) de l'invention et une molécule de phosphatidylsérine ;
- la figure 8 est une représentation schématique des interactions entre les résidus ligands du domaine 1 de la présente invention de l'annexine V humaine représenté sur la figure 6b, et une molécule de phosphatidylsérine en présence d'un atome de calcium ;
- les figures 9A et 9B sont des photographies de gels de polyacrylamide qui illustrent la fixation de l'annexine V et de certains de ses mutants sur des membranes constituées de phosphatidylcholine et de phosphatidylsérine (surnageant S2).

### EXEMPLES

### Exemple 1 : Expression et purification des peptides de séquences ID n°1 et ID n°2 de la présente invention

Les séquences ID n°1 et ID n°2 des annexines I et V ont été préparées par surexpression dans *E. Coli* selon le même protocole que celui qui a été décrit par F. Cordier-Ochsenbein et al. dans J. Mol. Biol. 279, 1177-1185.

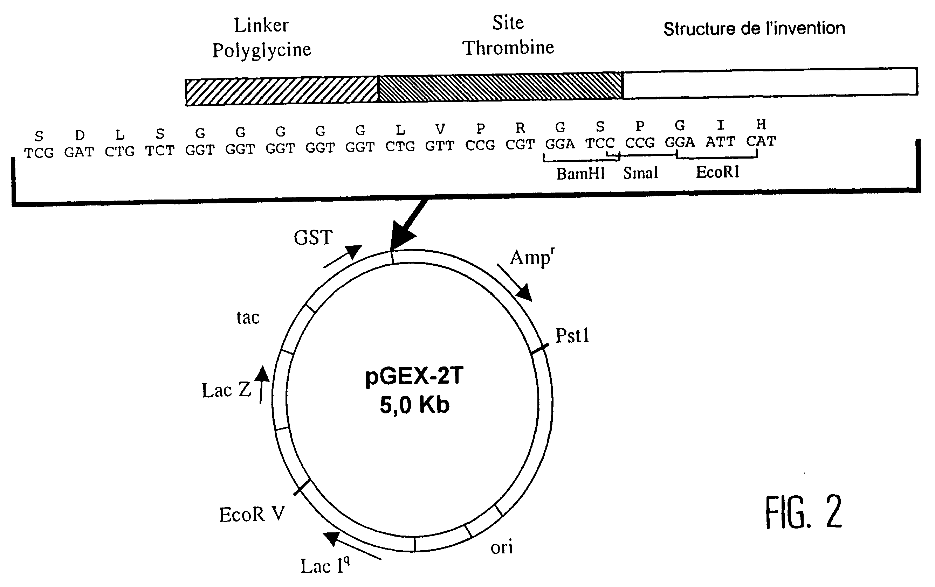

Le cDNA de ces séquences d'annexines a été préparé en utilisant la PCR à partir du cDNA des annexines correspondantes. Le cDNA a été inséré dans le vecteur pGEX-2T (Smith & Jonhson, 1998). La figure 2 est un schéma illustrant l'insertion du cDNA dans le vecteur. L'absence de mutations induites par la PCR a été contrôlée par séquençage. La production du peptide est effectuée en utilisant la souche *E. Coli* BL21 contenant le vecteur d'expression décrit plus haut. Après induction par l'isopropylthiogalactopyranoside (IPTG, 100 µm) jusqu'à une densité optique de 1 à 600 nm, la pousse est continuée jusqu'à ce qu'un plateau soit atteint, c'est-à-dire pendant environ 3 heures. Après centrifugation, les bactéries sont resuspendues dans le tampon de lyse comprenant 50 mM Tris-HCl, pH 8, 10 mM EDTA, 500 mM NaCl, 5% (v/v) glycérol, 1% (v/v) Triton X100, 1 mM dithiothréitol (DTT), 1 mM phénylméthylsulfonyl fluoride (PMSF) et 20 µg/ml aprotinine.

La purification a été effectuée de la façon suivante : après sonification et centrifugation à 10000 g, le surnageant contenant les protéines solubles est incubé avec des billes de glutathion/agarose permettant la liaison spécifique à ces billes de la protéine de fusion GST-domaine. Après lavage avec une solution contenant 1 M NaCl, 50 mM Tris-HCl à pH 8, 70 unités de thrombine par litre de culture sont ajoutés et la séquence est éluée.

La séquence est alors purifiée sur une colonne proRPC (marque de commerce) de type 16/10, fournie par la société Pharmacia en utilisant un système FPLC et un gradient linéaire d'eau de qualité Millipore (marque de commerce) contenant 0,1% (v/v) d'acide trifluoroacétique TFA, et d'acétonitrile contenant 0,1% de TFA. La vitesse d'écoulement est ajustée à 2,5 ml/minute. La séquence est ensuite lyophilisée. Le rendement final est d'environ 8 mg de séquence par litre de culture.

### Exemple 2 : Stabilité de la séquence ID n°1 de l'annexine 1

Différentes expériences montrent que cette séquence constitue une protéine de repliement stable.

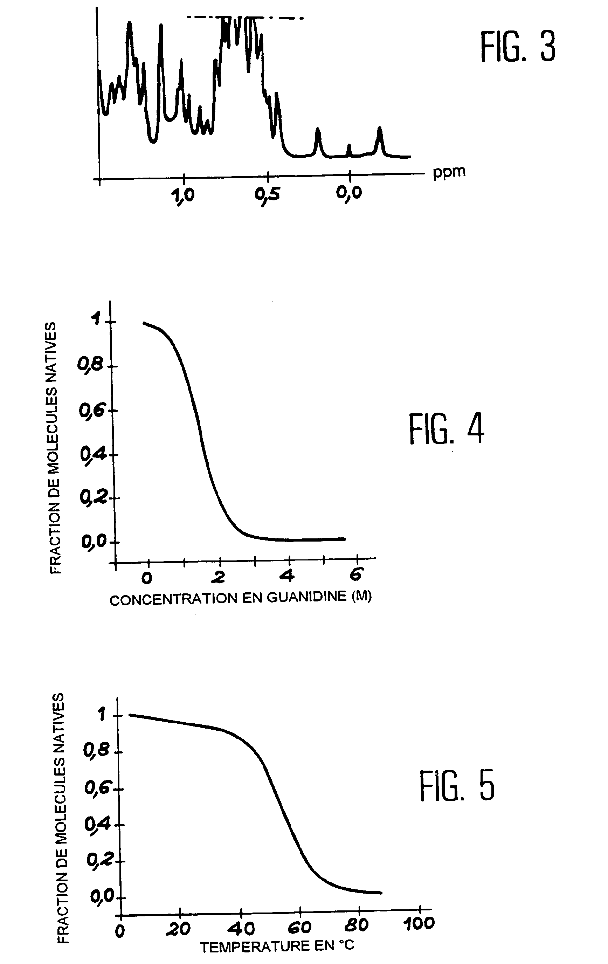

La figure 3 montre un spectre 1D de RMN ¹H du proton de la séquence ID n°1 isolée de l'annexine 1, en solution aqueuse. La dispersion des fréquences de résonance et la présence de résonances à des déplacements chimiques inférieurs à 0 ppm indiquent clairement que cette séquence est fortement structurée. De plus, les données de déplacement chimique des protons α révèlent la présence de 5 hélices conformément à la structure cristallographique.

La figure 4 montre la dénaturation coopérative du domaine 1 de l'annexine I issu de la séquence ID n°1 par le chlorure de guanidinium, qui est un dénaturant classique et la figure 5 montre la dénaturation coopérative de la séquence par la température.

Des données analogues sont obtenues pour les autres séquences décrites précédemment et démontrent que certaines séquences d'annexine se comportent comme de petites protéines de stabilité normale, utilisables directement, ou comme plate-forme, pour l'ingénierie de composés fonctionnels nouveaux.

### Exemple 3-1 : Rôle essentiel du domaine 1 de l'annexine V issu de la séquence ID n°2 dans la liaison de l'annexine V aux membranes

Des expériences de liaison de l'annexine V à des systèmes membranaires modèles ainsi que des expériences d'inhibition de la protéine kinase c (PKC) *in vitro*, et de la phospholipase A₂ (PLA₂) cytoplasmique (cPLA₂) *in vivo* démontrent le rôle essentiel joué par le domaine 1 dans cette liaison aux membranes.

On prend ici comme exemple le cas de l'inhibition de la cPLA₂. L'inhibition de l'activité phospholipasique par l'annexine V résulte de la déplétion du substrat lipidique commun à ces deux protéines. Divers mutants de l'annexine V ont été construits pour éliminer de façon sélective dans un ou plusieurs domaines la capacité de lier le calcium, c'est-à-dire les phospholipides. La mutation consiste à remplacer le ligand bidentate du calcium, Glu ou Asp, d'une séquence de la présente invention par un résidu non liant, Gln ou Asn respectivement. Douze mutants ont ainsi été construits et purifiés : M1, M2, M3, M4, M1M2, M1M3, M1M4, M2M3, M1M2M3, M1M2,M4, M2M3M4 et M1M2M3M4, le chiffre indiquant le domaine pour lequel la capacité de lier le calcium a été supprimée. L'ensemble des résultats montre que l'activité phospholipasique de la PLA₂ cellulaire, mesurée par le taux de relarguage d'acide arachidonique, dépend fortement de la présence du site calcium dans le domaine 1 et à un moindre degré dans le domaine 4. La suppression des sites calcium dans les domaines 2 et 3 n'a quasiment aucun effet sur l'inhibition de l'activité phospholipasique de la cPLA₂. (Mira et al. J. Biol. Chem. 1997, 272:10474-10482 ; Dubois et al. Biochem. J. 1998, 330:1277-1282).

Le tableau (I) suivant regroupe certains résultats de cet exemple, et montre le pourcentage de diminution de la liaison des mutants de l'annexine V aux phospholipides par rapport à l'annexine V sauvage.

| Annexine V sauvage | M1 | M2 | M3 | M4 | M1M2M3 | M1M2M4 |
|---|---|---|---|---|---|---|
| 0 | 79±6 | 38±4 | 47±9 | 38±6 | 98±1 | 85±7 |

Ce tableau (I) montre la liaison aux membranes de l'annexine V et de ses mutants M1, M2, M3, M4, M1M2M3 et M1M2M4. Les résultats sont exprimés en pourcentage de diminution de la capacité de liaison par rapport à l'annexine V sauvage (valeur moyenne ± erreur standard). Pour les mutants M123 et M124, le taux de liaison résiduel n'est pas significatif.

### Exemple 3-2 : Résultats préliminaires concernant la liaison de l'annexine V et de divers mutants aux membranes modèles composées de phosphatidylcholine et de phosphatidylsérine

Les mutants suivants de l'annexine V humaine ont été préparés selon les méthodes décrites dans l'exemple 1 :
M1M2M3M4 : le site calcium principal, correspondant à la boucle AB, est supprimé dans tous les domaines par une mutation du ligand bidentate.
M2M3M4 : le site calcium principal des domaines 2,3 et 4 est supprimé par une mutation du ligand bidentate, celui du domaine 1 subsiste.
M2M3M4-Arg22Ala-Arg63Ser : suppression des ligands L2 et L3 du site PS de la présente invention.
M2M3M4-Arg22Ala-Arg63Ser-Lys29Ala-Asp68Ile/Phe/Trp : suppression de tous les ligands du site PS de la présente invention sauf ceux concernant le site calcium qui eux sont conservés.

La liaison aux membranes PC/PS des mutants de l'annexine V est alors comparée à celle de la forme sauvage selon le protocole suivant :

Un mélange homogène de PC/PS dans une proportion 80/20 est mis en suspension dans des solutions contenant des concentrations variables de calcium à 0, 30, 100, 1000 µM. Les diverses protéines sont ensuite introduites et incubées pendant quelques minutes. La suspension est ensuite centrifugée par ultracentrifugation à 90000 t/mn. Les membranes sédimentent au fond du tube. Le surnageant, appelé S1, est entièrement prélevé pour analyse ultérieure du contenu en protéine qui donnera l'information sur la quantité de protéine non liée à la membrane. Le culot de membrane est ensuite dispersé dans une solution contenant de l'EDTA en quantité suffisante pour le relargage des protéines, la liaison de l'annexine V étant réversible et dépendante du calcium. La suspension est de nouveau centrifugée et un second surnageant, appelé S2, est récupéré. L'analyse du contenu en protéine de S2 fournira l'information concernant la quantité des protéines qui étaient fixées à la membrane.

L'analyse des surnageants est effectuée par électrophorèse sur gel de polyacrylamide d'une façon classique qu'il est inutile de décrire ici.

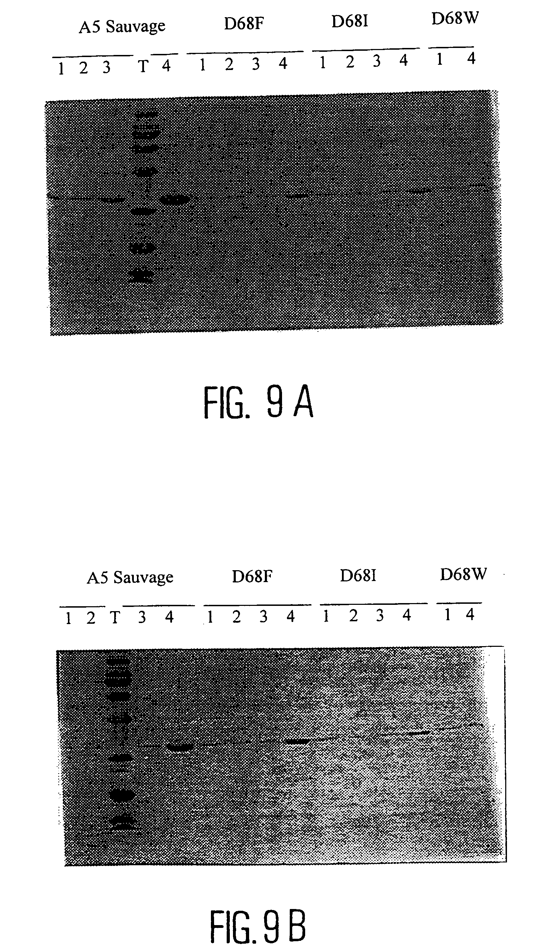

Les figures 9A et 9B en annexe montrent l'ensemble des résultats.

Sur cette figure :
Sauvage : A5 = annexine V
Mutants :
   D68F=M2M3M4-Arg22Ala-Arg63Ser-Lys29Ala-Asp68Phe
   D68I=M2M3M4-Arg22Ala-Arg63Ser-Lys29Ala-Asp68Ile
   D68W) M2M3M4-Arg22Ala-Arg63Ser-Lys29ala-Asp68Trp 1,2,3,4=concentration en calcium respectivement 0,30, 100, 1000 µM
T=témoins de masse moléculaire

Le comportement des mutants M1M2M3M4 et M2M3M4, comparé à celui de l'annexine V sauvage montre clairement que la liaison aux membranes en présence de calcium est quasi uniquement assurée par le domaine 1, c'est-à-dire qui contient le site PS revendiqué. Ce résultat confirme ceux présentés dans l'exemple 3-1 précédent.

Le comportement des mutants M2M3M4-Arg22Ala-Arg63Ser et M2M3M4-Arg22Ala-Arg63Ser-Lys29Ala-Asp68Ile/Phe/Trp montre que la liaison aux membranes est considérablement atténuée lorsque les ligands L2, L3, L4 et L5 sont supprimés. La liaison n'est cependant pas totalement supprimée dans la mesure où les ligands Lca5, Ca qui font partie du site calcium subsistent et permettent encore une liaison de la PS mais avec une affinité très diminuée.

### Exemple 4 : Utilisation de la structure chimique de la présente invention

Trois voies d'utilisation sont prévues : i) simple ingénierie des domaines pour satisfaire aux diverses exigences liées à leur utilisation comme outils de recherche, de diagnostic et de thérapeutique ; ii) "re-design" de la plate-forme qui constitue la topologie du domaine en une nouvelle plate-forme plus simple et synthétisable par voie chimique ou par génie génétique ; iii) remplacement de la plate-forme peptidique ou peptoïdique par une structure organique non-peptidique pour la fabrication d'un médicament. Dans les trois cas, il s'agit naturellement de conserver, voire d'améliorer, la localisation spatiale des fonctions de liaison avec les phospholipides, décrits plus haut.

### 1) Ingénierie des domaines d'annexines

Les domaines d'annexines de la présente invention constituent des plates-formes peptidiques. On entend par ingénierie la modification de la séquence des domaines par mutagénèse afin d'améliorer la stabilité générale de la molécule et de l'adapter aux conditions physico-chimiques imposées par l'utilisation, d'améliorer son affinité pour le ligand phospholipidique, et lui conférer une spécificité propre à chaque phospholipide. Il s'agit aussi de permettre l'introduction de différents marqueurs pour les différentes applications dont il est question plus loin. Nos connaissances actuelles sont largement suffisantes pour effectuer une telle ingénierie.

Les exemples d'un changement de propriété ont été illustrés dans l'exemple 4 Ils ont été obtenus par une technique classique de génie génétique en mutant les acides aminés impliquées.

### 2) "Re-design" des plates-formes peptidiques

Le "re-design" de la plate-forme consiste à redéfinir une architecture moléculaire, tout en gardant la topologie convenable des résidus impliqués dans la liaison au calcium et aux phospholipides. L'intérêt du "re-design" est de créer une plate-forme de séquence plus courte pouvant être produite par synthèse chimique. La synthèse d'un peptide de la taille d'un domaine est possible mais reste difficile. La réduction par deux du nombre de résidus, soit environ 35 résidus, rend par contre la synthèse couramment réalisable. Dans cette opération de "re-design", on conserve assez précisément la géométrie permettant des interactions avec le phospholipide, et notamment la disposition des résidus de la séquence annexine. Ces résidus sont ceux indiqués en gras sur les figures 6a à 6d pour les annexines (I) à (V).

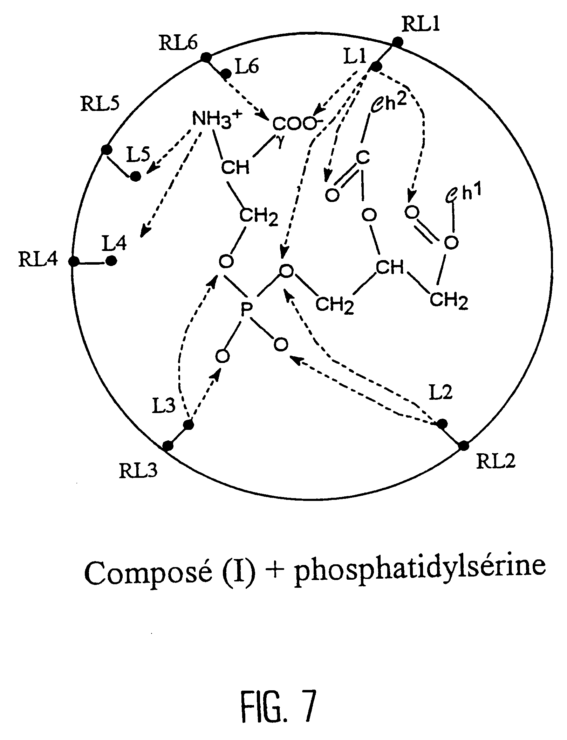

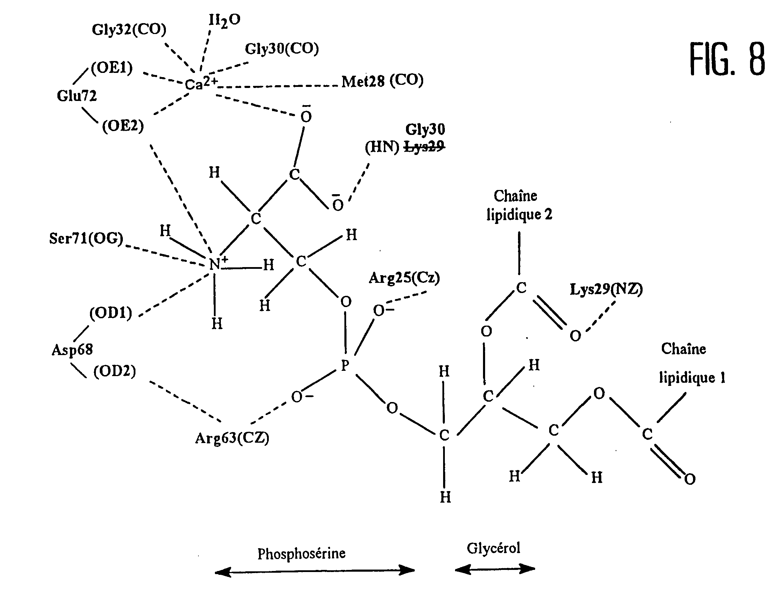

Cet ensemble comprend deux résidus basiques, généralement Arg-x-x-x-Lys, à la fin de l'hélice A du domaine concerné et une série de résidus acides, basiques et neutres, généralement Arg-x-x-x-x-Asp-x-x-Ser-Asp, situés dans l'hélice D. L'étude de la structure moléculaire montre, figures 7 et 8, que ces résidus sont parfaitement disposés pour lier une molécule de phosphatidylsérine. Le groupe carboxylate de ce lipide est lui-même lié à l'atome de calcium situé dans la boucle AB et désigné dans la suite par "site calcium AB".

La séquence :
**Arg**-x-x-x-**Lys**(hélice A)----**Arg**-x-x-x-x-**Asp**-x-x-**Ser-Asp**(hélice D)
   associée à celle du site calcium AB, constitue donc une séquence consensus pour la liaison de la phosphatidylsérine dans les composés de la présente invention. Pour généraliser, on désignera maintenant cette séquence par :
   **RL1**-x-x-x-**RL2**----**RL3**-x-x-x-x-**RL4**-x-x-**RL5-RL6**
   où RL1 à RL6 sont les résidus ligands essentiels dans la liaison de la phosphatidylsérine indiqués en gras dans les séquences des figures 6a à 6d et indiqué dans les composés de structure (I) à (VI). La séquence consensus du site calcium AB est la suite :
   ***Met***-Lys-***Gly***-x-***Gly***-Thr----***Asp***(ou ***Glu***)

Les ligands du calcium sont les groupes carboxyl peptidiques des résidus en italique (résidus de la boucle AB) sur la figure et les deux atomes d'oxygène du groupe carboxylate de la chaîne latérale du résidu Asp (ou Glu) de la fin de l'hélice D, dénommé aussi ligand bidentate. En généralisant ces ligands du calcium seront maintenant désignés par :
**RCa1**-**RL2**-**RCa2**-x-**RCa3**-Thr----(**RCa4RCa5**) ou **RL6**

Dans le cas de l'annexine, RCa4 et RCa5 constituent un seul et même résidu déjà identifié précédemment comme RL6.

Les données de distances interatomiques entre les résidus ligands sont donnés dans le tableau (II) suivant en référence à la figure 7 en annexe et les interactions précises domaine-calcium-phosphatidylsérine sont indiquées dans le tableau (III) suivant en référence à la figure 8 en annexe.

Sur la figure 8, Ch1 et Ch2 représentent la position d'éventuelles chaînes carbonnées du phospholipide. Ces chaînes peuvent être celles décrites, par exemple l'acide arachidonique.

Selon l'invention, la structure chimique peut être constituée de la façon suivante :
a) elle comporte en particulier au moins 6 résidus, dits résidus ligands, nommés RL1 à RL6 et dont la nature est la suivante :
RL1 = Arg ou Lys ou Orn
RL2 = Arg ou Lys ou Orn
RL3 = Arg ou Lys ou Orn
RL4 = Asp ou Glu
RL5 = Ser ou Thr ou Asp ou Glu
RL6 = Arg ou Lys ou Orn

b) les carbones α des résidus ligands RL1 à RL6 sont disposés dans l'espace de manière à ce que les chaînes latérales soient directement accessibles aux phospholipides,
c) les carbones α des résidus ligands RL1 à RL6 sont disposés selon le tableau II de distances suivant :

| Carbone α | RL2 | RL3 | RL4 | RL5 | RL6 |
|---|---|---|---|---|---|
| RL1 | 0,45 à 0,65 | 0,7 à 1,2 | 0,7 à 1,0 | 0,85 à 1,15 | 0,65 à 0,95 |
| RL2 | | 0,5 à 1,05 | 0,8 à 1,2 | 1,2 à 1,7 | 0,9 à 1,4 |
| RL3 | | | 0,5 à 0,8 | 1,0 à 1,3 | 1,2 à 1,7 |
| RL4 | | | | 0,45 à 0,75 | 0,7 à 1,2 |
| RL5 | | | | | 0,4 à 1,2 |

d) les chaînes latérales des résidus ligands RL1 à LR6 permettent d'établir un réseau de liaisons hydrogène avec la phosphatidylsérine selon le schéma où les flèches → désignent au moins une liaison hydrogène, figure 8, dans le sens donneur vers accepteur et L1 à L6 désignent les ligands de la phosphatidylsérine selon la liste suivante :
L1 = NZLYs ou CZArg de LR1
L2 = NZLys ou CZArg de LR2
L3 = NZLys ou CZArg de LR3
L4 = CGAsp ou CDGlu de LR4
L5 = CB de Ser ou Thr ou CG de Asp ou CD de Glu de LR5
L6 = NZLys ou CZArg de LR6
HN = H
NZ = N zeta
CZ = C zeta
OD = O delta
OG = O gamma
OE = O epsilon
où les distances entre les ligands L1 à L6 et les atomes de la phosphatidylsérine sont données dans le tableau III suivant :
Distances nm x 10

| | N | Cβ | Cγ | O1 | O2 | O3 | O4 | Cl chaîne Chl | Cl chaîne Ch2 |
|---|---|---|---|---|---|---|---|---|---|
| L1 | 0,35 à 0,65 | 0,3 à 0,5 | 0,25 à 0,45 | 0,2 à 0,35 | 0,25 à 0,5 | 0,35 à 0,6 | 0,2 à 0,35 | 0,4 à 0,7 | 0,5 à 0,8 |
| L2 | 0,55 à 0,85 | 0,45 à 0,75 | 0,45 à 0,75 | 0,4 à 0,6 | 0,2 à 0,4 | 0,4 à 0,6 | 0,25 à 0,45 | 0,7 à 1,1 | 0,7 à 1,1 |
| L3 | 0,4 à 0,6 | 0,4 à 0,6 | 0,45 à 0,75 | 0,4 à 0,6 | 0,2 à 0,4 | 0,2 à 0,35 | 0,25 à 0,5 | 0,7 à 1,1 | 0,6 à 1,0 |
| L4 | 0,25 à 0,45 | 0,3 à 0,5 | 0,35 à 0,55 | 0,55 à 0,85 | 0,5 à 0,75 | 0,4 à 0,65 | 0,4 à 0,6 | 0,8 à 1,2 | 0,8 à 1,2 |
| L5 | 0,25 à 0,5 | 0,45 à 0,65 | 0,5 à 0,75 | 0,65 à 0,95 | 0,65 à 0,95 | 0,5 à 0,8 | 0,5 à 0,9 | 0,8 à 1,2 | 0,6 à 1,0 |
| L6 | 0,3 à 0,5 | 0,35 à 0,55 | 0,3 à 0,45 | 0,65 à 0,95 | 0,7 à 1,0 | 0,65 à 0,95 | 0,5 à 0,8 | 0,6 à 1,0 | 0,8 à 1,2 |

Pour le ligand L1, deux au moins des cinq distances indiquées dans ce tableau sont de préférence respectées.

### 3) Plate-forme organique

La troisième étape constitue l'étape ultime pour l'obtention d'un médicament facilement utilisable par voie orale. Il s'agit du remplacement de la plate-forme peptidique par une structure organique respectant la disposition spatiale des ligands phospholipidiques. Les ligands calciques et phospholipidiques ne sont plus des résidus aminoacides mais des fonctions chimiques reproduisant les interactions décrites plus haut.

Les structures organiques couramment utilisées en pharmacologie peuvent permettre de construire des plates-formes rigides capables de présenter un site de liaison pour le phospholipide selon l'invention. Ces structures peuvent être constituées par les techniques chimiques classiques connues de l'homme du métier, qu'il n'est pas nécessaire ici de rappeler.

### Exemple 5 :

De manière très avantageuse, l'utilisation d'une structure ou d'un asemblage de la présente invention peut se faire comme indiqué précédemment dans trois directions : recherche, diagnostic et thérapeutique.

### 1) la recherche

Pour ces expériences, il convient de coupler une structure de la présente invention à une molécule de marquage permettant une détection. Ces molécules de marquage peuvent être celles précitées par exemple des molécules fluorescentes, un système avidine-biotine, des radioéléments, et de manière générale, celles couramment utilisées.

### 2) le diagnostic

Les structures chimiques et assemblages de la présente invention peuvent comme indiqué précédemment être utilisés pour la détection "in vitro" de pathologies impliquant l'apparition de charges négatives à la surface des cellules et la libération dans le sang de microvésicules : par exemple les troubles de la coagulation, les pathologies inflammatoires aiguës, etc...

Ils peuvent aussi être couplés à des radioéléments à vie courte et détection "*in vivo*" de la localisation des zones thrombotiques lors d'accidents vasculaires de toute sorte, en particulier cérébraux, utilisant des systèmes d'imagerie.

Ils peuvent aussi être couplés à des composés paramagnétiques, par exemple un complexe de gadolinium, et détection "*in vivo*" de la localisation des zones thrombotiques lors d'accidents vasculaires de toute sorte, en particulier cérébraux, utilisant l'imagerie par résonance magnétique (IRM).

Les couplages précités peuvent être réalisés par les techniques classiques de chimie organique connues de l'homme du métier, qu'il n'est pas utile ici de rappler.

### 3) le médicament

Les structures et assemblages de la présente invention peuvent être utilisés en tant que tels pour fabriquer un médicament utilisable pour un traitement ou pour une prophylaxie car ils possèdent des propriétés anticoagulante, antithrombolytique et anti-inflammatoire intrinsèques.

Les assemblages selon l'invention permettent d'effectuer un tapissage des surfaces cellulaires, capable d'interdire l'accès de composés impliqués dans les étapes primaires de la coagulation sanguine et les phénomènes inflammatoires à ces surfaces.

Les structures et assemblages de la présente invention peuvent également être utilisés pour le ciblage de molécules à un site du thrombus, de l'inflammation, ou vers une zone tumorale.

Dans cette utilisation, les structures et assemblages de la présente invention sont couplés à une molécule qui a une action thrombolytique, à une molécule qui a une action anti-inflammatoire, ou à une molécule qui a une action anti-tumorale respectivement.

Les structures et assemblages de la présente invention peuvent donc être utilisés par exemple pour fabriquer un médicament utilisable dans le traitement et la prophylaxie de la thrombose. Le couplage des structures et assemblages à des molécules à action thrombolytique permet un ciblage de ces dernières vers les zones thrombogènes. Les molécules thrombolytiques telles que les streptokinases, les urokinases, et les activateurs du plasminogène peuvent être utilisées.

Les structures et assemblages de la présente invention peuvent aussi être utilisés couplés à une molécule ayant une action anti-inflammatoire pour fabriquer un médicament utilisable par exemple par voie locale ou par voie orale dans des pathologies aiguës comme l'asthme, la RCH, le Crohn, le choc septique, les maladies du collagène et l'arthrite.

Les structures et assemblages de la présente invention peuvent aussi être utilisés couplés à une molécule ayant une action antitumorale. Ce couplage permet de cibler ces dernières molécules vers des zones présentant des charges négatives telles que des tumeurs possédant des foyers cellules apoptotiques, des tumeurs inflammatoires, etc...

Les structures et assemblages de la présente invention peuvent également être utilisés pour fabriquer un matériau de recouvrement de biomatériaux susceptibles d'être thrombogènes. Un biomatériau thrombogène ainsi recouvert perd ses propriétés thrombogènes. Le biomatériau thrombogène peut être par exemple une valve cardiaque.

L'invention propose d'utiliser une structure chimique dérivée des protéines de la famille des annexines et leurs domaines isolés, modifiés ou non, capables de se lier réversiblement aux effecteurs lipidiques tel que les phosphatidylsérines, les acides phosphatidiques, les phosphatidyléthanolamines et les phosphatidylinositophosphates. Il s'agit de fournir un ensemble de composés protéiques, peptidiques, peptoïdes et organiques dont la propriété pricnipale est de reconnaître spécifiquement l'apparition des signaux lipidiques à la surface des membranes cellulaires en relation avec le fonctionnement normal ou pathologique des tissus. Les pathologies spécialement visées par l'invention sont : (i) les troubles de la coagulation sanguine, (ii) les phénomènes d'apoptose consécutifs à l'action de composés chimiques, d'effets physiques comme les radiations ionisantes, d'effets biologiques comme ceux liés à la formation ou la nécrose des tissus cancéreux, outre les phénomènes normaux d'apoptose, (iii) les pathologies inflammatoires aiguës et (iv) les troubles associés aux relations entre les cellules et la matrice extra-cellulaire et notamment le collagène.

Outre l'ingénierie complète des annexines entières, un des aspects de l'invention est l'utilisation de domaines et de modules covalents d'annexines soit directement soit comme plate-forme pour l'ingénierie de composés peptidiques fonctionnels. Il s'agit donc d'utiliser ces domaines et modules soit sous leur forme naturelle, soit modifiés par les voies de la mutagénèse ou de la chimie, pour en faire des composés répondant aux critères biologiques exposés dans le paragraphe précédent.

De par leur faible taille, ces domaines peuvent être facilement associés à d'autres protéines soit pour former des protéines chimères multifonctionnelles, soit pour introduire un mécanisme de régulation par des effecteurs autres que les phospholipides de signalisation. De plus, l'invention se propose de redéfinir, par les méthodes de l'ingénierie des protéines, la spécificité des domaines pour les différents lipides de signalisation évoqués plus haut.

L'invention propose enfin de reconstruire ces domaines, par de *novo* design, pour en faire des composés de taille plus restreinte et accessible à la synthèse peptidique et en particulier à l'introduction de résidus aminoacides non naturels dans le but d'augmenter la durée de vie de ces composés dans l'organisme.

## Revendications

1. Structure peptidique ayant une affinité pour un phospholipide, **caractérisée en ce qu'**elle est constituée d'un peptide de formule (VII) suivante :
X-RL1-N¹-RL2-M-RL3-N²-RL4-N³-RL5-RL6-Y
dans laquelle N¹ à N³ représentent chacun indépendamment de 1 à 4 acides aminés choisis indépendamment, naturels ou non naturels, et dans laquelle M est un peptide constitué de 33 acides aminés naturels ou non naturels ; dans laquelle RL1, RL2, RL3 et RL6 sont choisis indépendamment parmi Lys, Arg ou Orn ; RL4 est choisi indépendamment parmi Asp ou Glu ; et RL5 est choisi indépendamment parmi Ser, Thr, Asp ou Glu, et
dans laquelle X est choisi parmi TPAQFDADEL (résidus 114-123 de SEQ ID n°1), DERADAETL (résidus 16-24 de SEQ ID n°2), PPAVFDAKQL (résidus 90-99 de SEQ ID n°3), NAMEDAQTL (résidus 15-23 de SEQ ID n°4) et PTVLYDVQELQ (résidus 86-95 de SEQ ID n°5) et
Y est choisi parmi TSGDFRNALLSLAKG (résidus 172-186 de SEQ ID n°1), LTGKFEKLIVALMKPSRLY (résidus 73-91 de SEQ ID n°2), TSGDFRKALLTLADG (résidus 148-162 de SEQ ID n°3), LSGNFEQVIVGMMT (résidus 72-85 de SEQ ID n°4) et TSFMFQRVLVSLSAGG (résidus 145-160 de SEQ ID n°5).

2. Structure selon la revendication 1, dans laquelle N¹ représente trois acides aminés, N² représente quatre acides aminés, et N³ représente deux acides aminés.

3. Structure selon la revendication 1, dans laquelle la molécule de formule (VII) est une séquence peptidique choisie parmi la séquence peptidique allant de Thr114 à Gly186 dans la séquence ID n°1 présentée sur la figure 6a, la séquence peptidique allant de Asp16 à Tyr91 dans la séquence ID n°2 présentée sur la figure 6b, la séquence peptidique allant de Pro90 à Gly162 dans la séquence ID n°3 présentée sur la figure 6c, la séquence allant de Asn15 à Thr85 dans la séquence ID n°4 présentée sur la figure 6d, la séquence allant de Pro86 à Gly160 dans la séquence ID n°5 présentée sur la figure 6, ou une séquence modifiée de ces séquences dans laquelle RL1, RL2, RL3 et RL6 sont choisis indépendamment parmi Lys, Arg ou Orn, RL4 est choisi indépendamment parmi Asp ou Glu, et RL5 est choisi indépendamment parmi Ser, Thr, Asp ou Glu.

4. Structure peptidique cyclique ayant une affinité pour un phospholipide chargé négativement, **caractérisée en ce qu'**elle est constituée d'une séquence peptidique de formule (VIII) suivante : 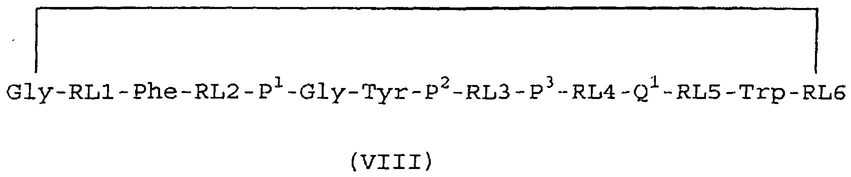 dans laquelle RL1 et RL6 sont choisis indépendamment parmi Lys, Orn et Arg ; RL2 et RL3 sont Arg ; RL4 et RL5 sont choisis indépendamment parmi Asp et Glu ;
dans laquelle P¹, P² et P³ sont choisis indépendamment parmi Ser et Thr ; dans laquelle Q¹ est choisi parmi Gly et Met.

5. Structure peptidique selon l'une quelconque des revendications 1 à 4, comprenant en outre un site calcium où l'ion calcium complexé par ce site constitue un des ligands du phospholipide chargé négativement.

6. Structure selon l'une quelconque des revendications précédentes, ladite structure ayant une affinité pour un phospholipide choisi parmi une phosphatidylsérine, une phosphatidyléthanolamine, un phosphatidylinositol, un acide phosphatidique, et un cardiolipide.

7. Assemblage ayant une affinité pour un phospholipide, **caractérisé en ce qu'**il comprend au moins deux structures peptidiques définies dans les revendications 1 à 6, identiques ou différentes, lesdites structures étant liées.

8. Assemblage selon la revendication 7, dans lequel au moins une des structures peptidiques est une des structures peptidiques définies dans les revendications 1 à 6.

9. Procédé de fabrication d'une structure peptidique définie dans l'une quelconque des revendications 1 à 6 précédentes, **caractérisé en ce qu'**il comprend les étapes de préparation d'un cDNA
comprenant une séquence de base codant pour ladite structure peptidique, d'insertion du cDNA dans un vecteur d'expression approprié, de transformation d'une cellule hôte appropriée pour une réplication du plasmide et la fabrication de ladite structure par traduction dudit cDNA.

10. Procédé selon la revendication 9, dans lequel le vecteur est un plasmide.

11. Procédé selon la revendication 9, dans lequel le vecteur est le vecteur pGEX-2T.

12. Procédé selon la revendication 9, 10 ou 11, dans lequel la cellule hôte appropriée est *E. Coli.*

13. Structure peptidique telle que définie dans les revendications 1 à 6 pour utilisation comme médicament.

14. Assemblage tel que défini dans la revendication 7 ou 8 pour utilisation comme médicament.

15. Structure peptidique selon la revendication 13 ou assemblage selon la revendication 14, caractérisé(e) en ce que ledit médicament est choisi parmi un médicament destiné au traitement d'une thrombose, un médicament destiné au traitement d'une tumeur, un médicament ayant une action anti-inflammatoire.

16. Utilisation d'une structure telle que définie dans les revendications 1 à 6 pour la fabrication d'un matériau de recouvrement d'un biomatériau thrombogène.

17. Composé de marquage **caractérisé en ce qu'**il comprend une structure telle que définie dans les revendications 1 à 6 couplée à une molécule de marquage.

18. Composé de marquage **caractérisé en ce qu'**il comprend un assemblage tel que défini dans la revendication 7 ou 8 couplé à une molécule de marquage.

19. Composé selon la revendication 17 ou 18 dans lequel la molécule de marquage est choisie parmi une molécule fluorescente, le complexe avidine-biotine, un radioélément, et un composé paramagnétique.

20. Trousse de diagnostic comprenant un composé selon l'une quelconque des revendications 17 ou 18.

21. Trousse de diagnostic selon la revendication 20, comprenant en outre un réactif adéquat permettant de détecter ladite molécule de marquage.

22. Trousse d'analyse et de détection de charges négatives à la surface de cellules, **caractérisée en ce qu'**elle comprend une structure selon l'une quelconque des revendications 1 à 6 couplée à un marqueur.

23. Trousse d'analyse et de détection de charges négatives à la surface de cellules, **caractérisée en ce qu'**elle comprend un assemblage selon la revendication 7 ou 8 couplé à un marqueur.

24. Trousse d'analyse et de détection de microvésicules dans le sang, **caractérisée en ce qu'**elle comprend une structure selon l'une quelconque des revendications 1 à 6 couplée à un marqueur.

25. Trousse d'analyse et de détection de microvésicules dans le sang, **caractérisée en ce qu'**elle comprend un assemblage selon la revendication 7 ou 8 couplé à un marqueur.

## Claims

1. Peptide structure with an affinity for a phospholipid, **characterized in that** it consists of a peptide with the following formula (VII):
X-RL1-N¹-RL2-M-RL3-N²-RL4-N³-RL5-RL6-Y (VII)
wherein N¹ to N³ each independently represent 1 to 4, independently selected, natural or non-natural, amino acids and wherein M is a peptide consisting of 33 natural or non-natural amino acids;
wherein RL1, RL2, RL3 and RL6 are independently selected from Lys, Arg or Orn; RL4 is independently selected from Asp or Glu; and RL5 is independently selected from Ser, Thr, Asp, or Glu, and
wherein X is selected from TPAQFDADEL (residues 114-123 of SEQ ID. n°1) , DERADAETL (residues 16-24 of SECT ID n°2), PPAVFDAKQL (residues 90-99 of SEQ ID n°3), NAMEDAQTL (residues 15-23 of SEQ ID n°4) and PTVLYDVQELQ (residues 86-95 of SEQ ID n°5) and
Y is selected from TSGDFRNALLSLAKG (residues 172-186 of SEQ ID n°1), LTGKFEKLIVALMKPSRLY (residues 73-91 of SEQ ID n°2), TSGDFRKALLTLADG (residues 148-162 of SEQ ID n°3), LSGNFEQVIVGMMT (residues 72-85 of SEQ ID n°4) and TSFMFQRVLVSLSAGG (residues 145-160 of SEQ ID n°5).

2. Structure according to claim 1, wherein N¹ represents three amino acids, N² represents four amino acids, and N³ represents two amino acids.

3. Structure according to claim 1, wherein the structure of formula (VII) is a peptide sequence selected from the peptide sequence from Thr114 to Gly186 in the sequence ID No.1 shown in Fig. 6a, the peptide sequence from Asp16 to Tyr91 in the sequence ID No.2 shown in Fig. 6b, the peptide sequence from Pro90 to Gly162 in the sequence ID No.3 shown in Fig. 6c, the sequence from Asn15 to Thr85 in the sequence ID No.4 shown in Fig. 6d, the sequence from Pro86 to Gly160 in the sequence ID No.5 shown in Fig. 6, or a modified sequence of these sequences in which RL1, RL2, RL3 and RL6 are independently selected from Lys, Arg, or Orn, RL4 is independently selected from Asp or Glu, and RL5 is independently selected from Ser, Thr, Asp or Glu.

4. Cyclic peptide structure with an affinity for a negatively charged phospholipid, **characterized in that** it consists of a peptide sequence of the following formula (VIII) : 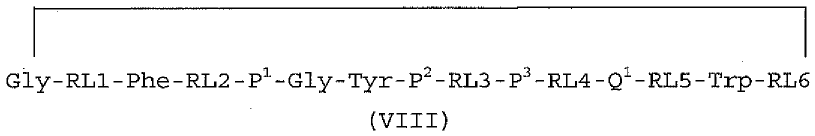 wherein RL1 and RL6 are independently selected from Lys, Orn and Arg; RL2 and RL3 are Arg; RL4 and RL5 are independently selected from Asp and Glu;
wherein P¹, P² and P³ are independently selected from Ser and Thr; wherein Q¹ is selected from Gly and Met.

5. Peptide structure according to any of claims 1 to 4, further comprising a calcium site where the calcium ion complexed by this site forms one of the ligands of the negatively charged phospholipid.

6. Structure according to any of the preceding claims, said structure having an affinity for a phospholipid selected from a phosphatidylserine, a phosphatidylethanolamine, a phosphatidylinositol, a phosphatidic acid, and a cardiolipid.

7. Assembly having an affinity for a phospholipid, **characterized in that** it comprises at least two identical or different peptide structures defined in claims 1 to 6, said structures being bound.

8. Assembly according to claim 7, wherein at least one of the peptide structures is one of the peptide structures defined in claims 1 to 6.

9. Method for producing a peptide structure as defined in any of the preceding claims 1 to 6, **characterized in that** it comprises steps consisting of preparing a cDNA comprising a base sequence coding for said peptide structure, inserting the cDNA in an appropriate expression vector, transforming an appropriate host cell for replicating the plasmid and producing said structure by translation of said cDNA.

10. Method according to claim 9, wherein the vector is a plasmid.

11. Method according to claim 9, wherein the vector is a pGEX-2T vector.

12. Method according to claim 9, 10 or 11, wherein the appropriate host cell is *E. Coli.*

13. Peptide structure as defined in claims 1 to 6 for use as a medicament.

14. Assembly as defined in claim 7 or 8 for use as a medicament.

15. Peptide structure according to claim 13 or assembly according to claim 14, **characterized in that** said medicament is selected from a medicament for treating a thrombosis, a medicament for treating a tumor, a medicament with an anti-inflammatory action.

16. Use of a structure as defined in claims 1 to 6 for producing a material for covering thrombogenic biomaterial.

17. Labelling compound **characterized in that** it comprises a structure as defined in claims 1 to 6 coupled with a labelling molecule.

18. Labelling compound **characterized in that** it comprises an assembly as defined in claim 7 or 8 coupled with a labelling molecule.

19. Compound according to claim 17 or 18, wherein the labelling molecule is selected from a fluorescent molecule, the avidin-biotin complex, a radioelement, and a paramagnetic compound.

20. Diagnosis kit comprising a compound according to any of claims 17 or 18.

21. Diagnosis kit according to claim 20, further comprising an adequate reagent enabling said labelling molecule to be detected.

22. Kit for analyzing and detecting negative charges at the surface of cells, **characterized in that** it comprises a structure according to any of claims 1 to 6, coupled with a tracer.

23. Kit for analyzing and detecting negative charges at the surface of cells, **characterized in that** it comprises an assembly according to claim 7 or 8, coupled with a tracer.

24. Kit for analyzing and detecting microvesicles in blood, **characterized in that** it comprises a structure according to any of claims 1 to 6, coupled with a tracer.

25. Kit for analyzing and detecting microvesicles in blood, **characterized in that** it comprises an assembly according to claim 7 or 8, coupled with a tracer.

## Patentansprüche

1. Peptidstruktur mit einer Affinität zu einem Phospholipid, **dadurch gekennzeichnet, dass** sie sich aus einem Peptid der folgenden Formel (VII) zusammensetzt:
X-RL1-N¹-RL2-M-RL3-N²-RL4-N³-RL5-RL6-Y
worin N¹ bis N³ jeweils unabhängig 1 bis 4 unabhängig ausgewählte, natürliche oder nicht natürliche Aminosäuren darstellen und worin M ein Peptid ist, das sich aus 33 natürlichen oder nicht natürlichen Aminosäuren zusammensetzt; worin RL1, RL2, RL3 und RL6 unabhängig aus Lys, Arg oder Orn ausgewählt sind; RL4 unabhängig aus Asp oder Glu ausgewählt ist und RL5 unabhängig aus Ser, Thr, Asp oder Glu ausgewählt ist und worin X aus TPAQFDADEL (Reste 114-123 der SEQ ID Nr. 1), DERADAETL (Reste 16-24 der SEQ ID Nr. 2), PPAVFDAKQL (Reste 90-99 der SEQ ID Nr. 3), NAMEDAQTL (Reste 15-23 der SEQ ID Nr. 4) und PTVLYDVQELQ (Reste 86-95 der SEQ ID Nr. 5) ausgewählt ist und Y aus TSGDFRNALLSLAKG (Reste 172-186 der SEQ ID Nr. 1), LTGKFEKLIVALMKPSRLY (Reste 73-91 der SEQ ID Nr. 2), TSGDFRKALLTLADG (Reste 148-162 der SEQ ID Nr. 3), LSGNFEQVIVGMMT (Reste 72-85 der SEQ ID Nr. 4) und TSFMFQRVLVSLSAGG (Reste 145-160 der SEQ ID Nr. 5) ausgewählt ist.

2. Struktur gemäß Anspruch 1, bei der N¹ drei Aminosäuren darstellt, N² vier Aminosäuren darstellt und N¹ zwei Aminosäuren darstellt.

3. Struktur gemäß Anspruch 1, bei der das Molekül der Formel (VII) eine Peptidsequenz ist, ausgewählt aus der von Thr114 bis Gly186 reichenden Peptidsequenz in der in Figur 6a dargestellten Sequenz ID Nr. 1, der von Asp16 bis Tyr91 reichenden Peptidsequenz der in Figur 6b dargestellten Sequenz ID Nr. 2, der von Pro90 bis Gly162 reichenden Peptidsequenz der in Figur 6c dargestellten Sequenz ID Nr. 3, der von Asn15 bis Thr85 reichenden Peptidsequenz der in Figur 6d darstellten Sequenz ID Nr. 4, der von Pro86 bis Gly160 reichenden Peptidsequenz der in Figur 6 dargestellten Sequenz ID Nr. 5 oder einer modifizierten Sequenz dieser Sequenzen, worin RL1, RL2, RL3 und RL6 unabhängig aus Lys, Arg oder Orn ausgewählt sind, RL4 unabhängig aus Asp oder Glu ausgewählt ist und RL5 unabhängig aus Ser, Thr, Asp oder Glu ausgewählt ist.

4. Cyclische Peptidstruktur, die eine Affinität zu einem negativ geladenen Phospholipid aufweist, **dadurch gekennzeichnet, dass** sie sich aus einer Peptidsequenz der folgenden Formel (VIII) zusammensetzt: 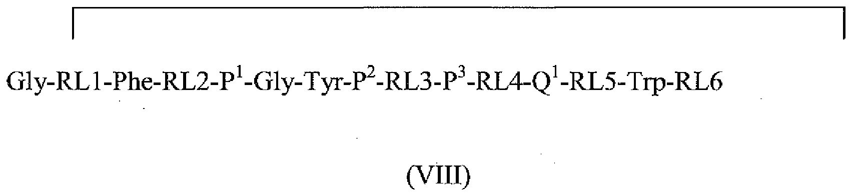 worin RL1 und RL6 unanhängig aus Lys, Orn und Arg ausgewählt sind; RL2 und RL3 Arg sind; RL4 und RL5 unabhängig aus Asp und Glu ausgewählt sind; worin P¹, P² und P³ unabhängig aus Ser und Thr ausgewählt sind und worin Q¹ aus Gly und Met ausgewählt ist.

5. Peptidstruktur gemäß einem der Ansprüche 1 bis 4, die außerdem eine Calcium-bindende Stelle umfasst, wo das durch diese Stelle komplexierte Calciumion einen der negativ geladenen Liganden des Phospholipids darstellt.

6. Struktur gemäß einem der vorangehenden Ansprüche, wobei diese Struktur eine Affinität zu einem Phospholipid aufweist, das aus einem Phosphatidylserin, einem Phosphatidylethanolamin, einem Phosphatidylinosit, einer Phosphatidsäure und einem Cardiolipid ausgewählt ist.

7. Verbund mit einer Affinität zu einem Phospholipid, **dadurch gekennzeichnet, dass** er wenigstens zwei in den Ansprüchen 1 bis 6 definierte, gleiche oder verschiedene Peptidstrukturen umfasst, wobei diese Strukturen verbunden sind.

8. Verbund gemäß Anspruch 7, bei dem wenigstens eine der Peptidstrukturen eine der in den Ansprüchen 1 bis 6 definierten Peptidstrukturen ist.

9. Verfahren zur Herstellung einer in einem der vorangehenden Ansprüche 1 bis 6 definierten Peptidstruktur, **dadurch gekennzeichnet, dass** es die Schritte der Herstellung einer cDNA, die eine für diese Peptidstruktur kodierende Basensequenz umfasst, der Inserierung der cDNA in einen geeigneten Expressionsvektor, der Transformation einer für die Replikation des Plasmids geeigneten Wirtszelle und der Herstellung dieser Struktur durch Transduktion dieser cDNA umfasst.

10. Verfahren gemäß Anspruch 9, wobei der Vektor ein Plasmid ist.

11. Verfahren gemäß Anspruch 9, wobei der Vektor der Vektor pGEX-2T ist.

12. Verfahren gemäß Anspruch 9, 10 oder 11, wobei die geeignete Wirtszelle E. coli ist.

13. Peptidstruktur wie in den Ansprüchen 1 bis 6 definiert zur Verwendung als Arzneimittel.

14. Verbund wie in Anspruch 7 oder 8 definiert zur Verwendung als Arzneimittel.

15. Peptidstruktur gemäß Anspruch 13 oder Verbund gemäß Anspruch 14, **dadurch gekennzeichnet, dass** dieses Arzneimittel aus einem zur Behandlung von Thrombose bestimmten Arzneimittel, einem zur Behandlung eines Tumors bestimmten Arzneimittel und einem Arzneimittel mit einer entzündungshemmenden Wirkung ausgewählt ist.

16. Verwendung einer wie in den Ansprüchen 1 bis 6 definierten Struktur zur Herstellung eines Materials zum Abdecken eines thrombogenen Biomaterials.

17. Markerverbindung, **dadurch gekennzeichnet, dass** sie eine wie in den Ansprüchen 1 bis 6 definierte, an ein Markermolekül gekuppelte Struktur umfasst.

18. Markerverbindung, **dadurch gekennzeichnet, dass** sie einen wie in Anspruch 7 oder 8 definierten, an ein Markermolekül gekuppelten Verbund umfasst.

19. Verbindung gemäß Anspruch 17 oder 18, bei der das Markermolekül aus einem fluoreszierenden Molekül, dem Avidin-Biotin-Komplex, einem Radioelement und einer paramagnetischen Verbindung ausgewählt ist.

20. Diagnosekit umfassend eine Verbindung gemäß einem der Ansprüche 17 oder 18.

21. Diagnosekit gemäß Anspruch 20 außerdem umfassend ein entsprechendes Reagenz, das den Nachweis des Markermoleküls erlaubt.

22. Kit zur Analyse und zum Nachweis negativer Ladungen auf der Oberfläche von Zellen, **dadurch gekennzeichnet, dass** er eine an einen Marker gekoppelte Struktur gemäß einem der Ansprüche 1 bis 6 umfasst.

23. Kit zur Analyse und zum Nachweis negativer Ladungen auf der Oberfläche von Zellen, **dadurch gekennzeichnet, dass** er einen an einen Marker gekuppelten Verbund gemäß Anspruch 7 oder 8 umfasst.

24. Kit zur Analyse und zum Nachweis von Mikrovesikeln im Blut, **dadurch gekennzeichnet, dass** er eine an einen Marker gekuppelte Struktur gemäß einem der Ansprüche 1 bis 6 umfasst.

25. Kit zur Analyse und zum Nachweis von Mikrovesikeln im Blut, **dadurch gekennzeichnet, dass** er einen an einen Marker gekuppelten Verbund gemäß Anspruch 7 oder 8 umfasst.
